# EUROPEAN PATENT SPECIFICATION

(11) **EP 2 703 452 B1**
(45) Date of publication and mention of the grant of the patent: **30.12.2015**
(21) Application number: 13192460.7
(22) Date of filing: 27.10.2011
(51) Int. Cl.: C09D 1/00, C09D 5/14, C09D 5/16

(54) **Method for making an anti-bacterial covering panel**
Verfahren zum Herstellen eines anti-bakteriellen Verkleidungspaneels
Procédé de fabrication d'un panneau de revêtement antibactérien

(30) Priority: 27.10.2010 IT BO20100644
(43) Date of publication of application: 05.03.2014
(62) Divisional of application: 11794854.7
(73) Proprietor: Next Technology Tecnotessile Societa' Nazionale di Ricerca R.L., 59100 Prato (PO) (IT)
(72) Inventor: Nesti, Solitario, 51035 SAN BARONTO (PISTOIA) (IT); Corsi, Leopoldo, 56038 PONSACCO (PISA) (IT); Lombardi, Luca, 51100 PISTOIA (IT); Fatarella, Enrico, 57034 CAMPO NELL'ELBA (LIVORNO) (IT)
(74) Representative: Firmati, Leonardo

(56) References cited:
- EP-A2- 1 288 391
- DATABASE WPI Week 200464 Thomson Scientific, London, GB; AN 2004-658505 XP002719148, -& JP 2004 262338 A (MITSUBOSHI BELTING LTD) 24 September 2004 (2004-09-24)
- DATABASE WPI Week 200463 Thomson Scientific, London, GB; AN 2004-646263 XP002719155, -& JP 2004 255665 A (TOUMEI KK) 16 September 2004 (2004-09-16)

## Description

### Technical Field

This invention relates to a method for making an anti-bacterial covering panel.

More specifically, the invention relates to the process for making an antibacterial panel used for covering rooms that must be kept under special hygiene conditions. For example, this invention is applicable in particular to ambulance sanitary compartments, medical and operating rooms, vans for the transportation of food products and food storage rooms, which are subject to particularly stringent requirements regarding bacterial charge reduction.

### Background Art

As is known, the sanitary compartments of ambulances are subject to disinfection and sanitisation procedures intended to keep bacterial propagation under control. In effect, the wall and floor coverings must be kept under optimum hygiene conditions at all times in order to prevent the formation and propagation of bacteria which are harmful to the health of patients and medical operators. Sanitisation and disinfection are carried out manually by personnel in charge of periodically cleaning all the floor and wall surfaces of the sanitary compartment with specific disinfecting substances.

Manual sanitisation and disinfection procedures do not, however, guarantee the total elimination of bacterial agents. Indeed, it should be borne in mind that manual cleaning, besides being time-consuming and labour intensive, is often inadequate for eliminating bacterial contamination in areas which are difficult to get at.

Also known in the prior art are devices, used mainly in operating rooms, for applying chemical sterilising substances in the room to be sanitised.

These devices are activated automatically and allow the sterilising substance to be sprayed in the operating room in order to sanitise the room thoroughly when it is not being used.

These devices, too, are not free of disadvantages, however.

In effect, the sterilising substances used are harmful for operators, especially if applied in the large quantities needed for effective sanitisation.

For that reason, after the sterilising substance has been applied, the room treated cannot be used for a certain length of time, necessary for the sterilising substance to be evacuated.

As a result, sanitising and bacterial charge reduction procedures are very long and require the treated room to be left unused for some time after treatment.

### Aim of the Invention

In this context, the main technical purpose of this invention is to provide a method for making an anti-bacterial covering panel which allows the above mentioned disadvantages to be overcome.

More specifically, this invention has for an aim to provide a method for making an anti-bacterial covering panel which can be used in any room, which allows the relative bacterial charge to be eliminated efficiently and in a very limited time, and whose action is continuous over time.

A further aim of the invention is to provide a method for making an anti-bacterial covering panel which is inexpensive, and structurally simple and which allows the bacterial charge to be eliminated without requiring any particular operations to be performed manually.

The technical purpose and aims specified are achieved by a method for making the anti-bacterial covering panel, comprising the technical features described in one or more of the accompanying claims from 1 to 8.

An example of a prior art method is disclosed in document JP2004262338, which describes the application of a single layer of photocatalytic particles to the surface of a mold wherein an olefin resin is injected by injection molding in order to define an epidermis of a polypropylene core.

In other words, this document only discloses the application of a single photocatalytic layer immediately followed by the injection (in a closed mold) of the epidermis and core material.

### Brief Description of the Drawings

Further features and advantages of the invention are more apparent in the nonlimiting description which follows of a preferred and hence non-exclusive embodiment of a method for making an anti-bacterial covering panel, as illustrated in the accompanying drawings, in which:
- Figure 1 is a schematic perspective view of an anti-bacterial covering panel and shows in detail the layers making up the panel and a portion of the mould used to make the panel;
- Figure 2 is a schematic perspective view of an anti-bacterial covering panel according to a second embodiment;
- Figure 3 is a side elevation view, partly in cross section, showing an ambulance whose interior is covered by anti-bacterial panels; and
- Figure 4 is a side elevation view, partly in cross section, showing a goods transport vehicle whose interior is covered by anti-bacterial panels.

### Detailed Description of Anti-Bacterial Covering Panels

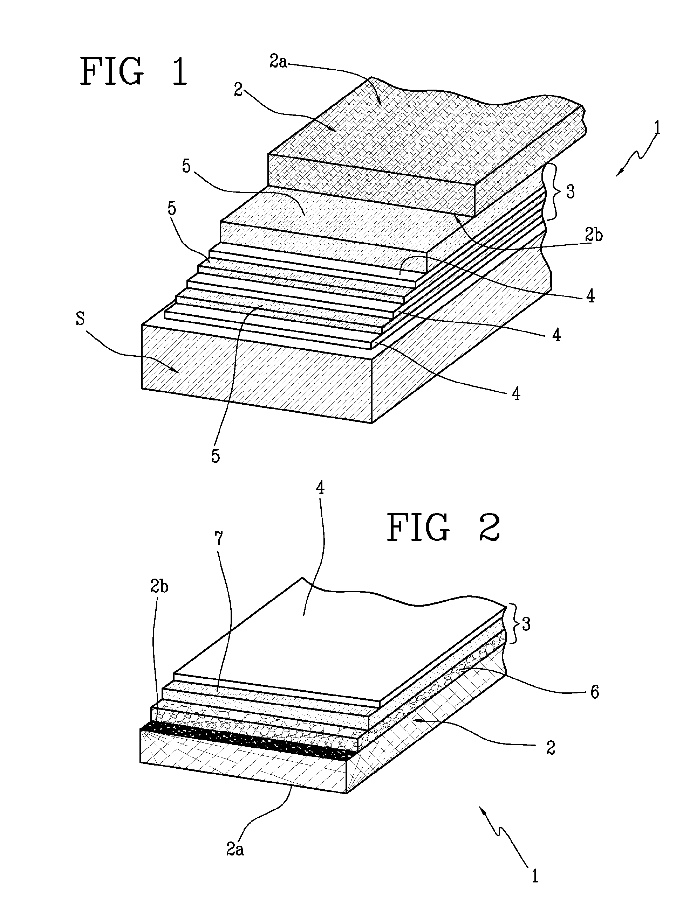

With reference to the accompanying drawings, an anti-bacterial covering panel is denoted in its entirety by the numeral 1. In Figures 1 and 2, the thicknesses of the layers making up the panel 1 are illustrated by way of example only and the thicknesses of the layers shown must not therefore be considered realistic.

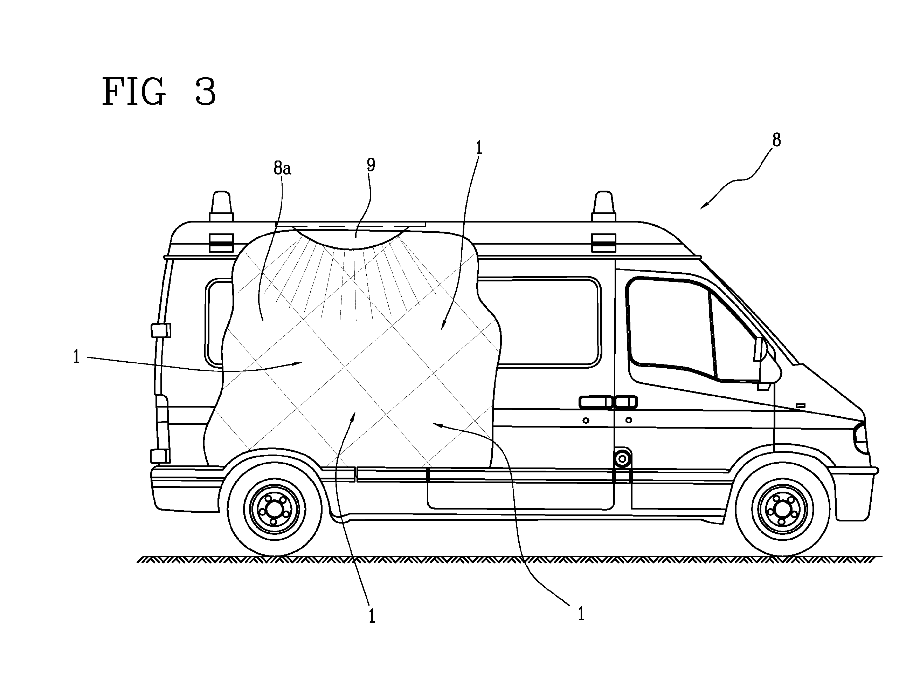

The panel 1 comprises a supporting layer 2 designed to be connected to a surface to be covered, for example the floor or side wall of the sanitary compartment of an ambulance (as illustrated in Figure 3) or of a goods vehicle, for example for transporting food products.

More specifically, the supporting layer 2 has a specified stiffness to confer mechanical strength to the panel 1 as a whole and comprises a first surface 2a adapted to be connected or coupled to a surface to be covered. On the side of it opposite the first surface 2a, the supporting layer 2 has a second surface 2b which a composite material 3 is associated with.

The composite material 3 is positioned outside in view at the second surface 2b of the supporting layer 2 and comprises at least one layer 4 of particles of photocatalytic material.

Preferably the photocatalytic material is titanium dioxide.

Preferably, the layer 4 of particles of photocatalytic material is placed in view, that is to say, it constitutes an outside surface of the composite material 3. This enhances the efficacy of the photocatalytic action because it is not screened by other layers of material.

More in detail, the layer 4 of particles of photocatalytic material consists of nanoparticle titanium dioxide. Advantageously, the layer 4 of particles of photocatalytic material defines a nanometer thickness.

Preferably, the layer 4 is obtained from a solution of nanoparticle titanium dioxide.

Still more preferably, the titanium dioxide solution is an alcoholic solution.

According to preferred embodiments the solution, whether alcoholic or non-alcoholic, contains other chemical substances with an antiseptic and/or disinfecting action.

Advantageously, by way of an example, one of the substances with an antiseptic and/or disinfecting action is benzoic acid.

The anti-bacterial panel 1 described below, made according to the first embodiment illustrated in Figure 1, is used preferably to cover the interior surfaces of the sanitary compartments 8a of ambulances 8.

In this embodiment, the supporting layer 2 is made preferably of fibreglass.

More specifically, as illustrated in Figure 1, the composite material 3 comprises a plurality of superposed layers 4 of particles of photocatalytic material. Each layer 4 of particles of photocatalytic material has a layer 5 of resin distributed on it. Preferably, the resin is polyester.

In this situation, the layers 4 of particles of photocatalytic material are spaced out from each other by the resin layers 5.

In other words, the resin layers 5 are interposed between the layers 4 of photocatalytic material.

Preferably, as illustrated in Figure 1, on the second surface 2b of the supporting layer 2 there are three layers 4 of particles of photocatalytic material which are alternated with, and spaced out by, the resin layers 5.

A further resin layer 5, thicker than the other two resin layers 5 which space out the three layers 4 of particles of photocatalytic material, is positioned on the innermost layer 4 of particles of photocatalytic material (the layer 4 closest to the supporting layer 2).

Preferably, each resin layer 5 is a gelcoat usually containing colour pigments to give the panel 1a certain aesthetic appearance.

Further, an antibacterial compound, preferably benzoic acid, may be dispersed in the gelcoat. The benzoic acid, added in a proportion of 4%, is first crushed with a roller and then applied by spraying.

An alternative antibacterial compound might be, for example, ammonium benzoate.

The anti-bacterial panel 1 described below, made according to the second embodiment illustrated in Figure 2, is used preferably to cover the floors of the sanitary compartments 8a of ambulances 8.

In this case, the supporting layer 2 is made of wood and is preferably, but not necessarily, 1 cm thick.

Further, the composite material 3 comprises at least one layer 6 of two-component resin which is interposed between the visible layer 4 of particles of photocatalytic material and the supporting layer 2.

More in detail, the layer 6 of two-component resin, preferably furnished with plastic chips to give the panel 1 a "grit effect", is distributed on the second surface 2b of the supporting layer 2.

A further layer 7 of two-component resin is applied by roller to the layer 6. The layer 4 of particles of photocatalytic material is applied to the outermost layer 7 of two-component resin. In this case, too, the layer 4 of particles of photocatalytic material consists of nanoparticle titanium dioxide. Advantageously, the layer 4 of photocatalytic material defines a nanometer thickness.

According to the first embodiment of Figure 1, the panel 1 described above in mainly structural terms is made by setting up a mould S to delimit a spatial area with the shape of the panel 1 to be obtained.

Preferably, the mould S is made of wood.

A releasing agent is first applied to the inside surface of the mould S and the solution of nanoparticle titanium dioxide is then sprayed on it.

Preferably, the releasing agent is wax or a wax-based product.

Next, the thin layer 4 of particles of photocatalytic material is applied by spraying. The sequence is repeated until there are three superposed layers 4 of particles of photocatalytic material spaced out by two resin layers 5.

A larger quantity of resin is then sprayed on the last layer 4 to be applied so as to form the thicker, innermost resin layer 5.

Next, the supporting layer 2 is applied to the last resin layer 5.

Once cross-linking has taken place, the mould S is removed to obtain the panel 1. The term cross-linking denotes the process whereby the material making up the supporting layer 2 hardens, this hardening process being due to the presence of suitable catalysing agents in the material.

According to the second embodiment of Figure 2, the layer 6 of two-component resin and plastic chips is applied directly to the second surface 2b of the supporting layer 2.

Once cross-linking has taken place, the next layer 7 of two-component resin, preferably transparent, is applied by roller.

Lastly, before cross-linking of the layer 7 occurs, the alcoholic solution of nanoparticle titanium dioxide is sprayed on.

With reference in particular to Figure 3, it should be noted that the panel 1 is used to cover the internal compartment 8a of an ambulance 8.

The drawing schematically illustrates a set of panels 1 placed side by side to cover a side wall of the compartment 8a. The anti-bacterial panels 1 described above may, however, be used to cover both the floor and any other surface inside the ambulance 8.

The sanitary compartment 8a also has a light source 9 for photocatalysis of the anti-bacterial covering panels 1.

Advantageously, the light source 9 designed to irradiate the anti-bacterial panels 1 is a lamp with an emission spectrum of between 290 and 780 nm and a power of between 10 and 500 W.

Preferably, the power of the lamp or lamps is about 250 W, with a maximum radiation of 380 nm.

In use, the photocatalytic action of the panels 1 in the ambulance 8 is started by the lamp 9. The lamp 9 thus activates the anti-bacterial property of the titanium dioxide contained in the panels 1. The anti-bacterial property thus depends on the length of time the panels are subjected to irradiation.

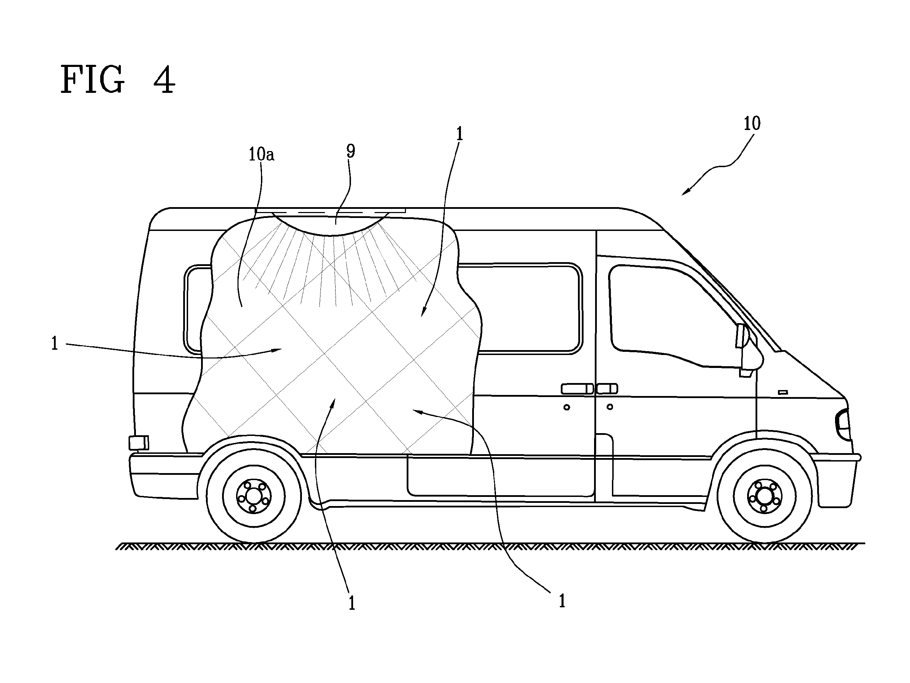

As illustrated in Figure 4, the panel 1 is used to cover the internal compartment 10a of a vehicle such as a van 10 used for the transportation of food products.

The drawing schematically illustrates a set of panels 1 placed side by side to cover a side wall of the compartment 10a. The anti-bacterial panels 1 described above may, however, be used to cover both the floor and any other surface inside the van 10.

The compartment 10a also has a light source 9 for photocatalysis of the anti-bacterial covering panels 1.

Advantageously, the use of the panels 1 in means for the transportation of food products makes it possible to guarantee better storage conditions and at the same time limits the risks of bacterial contamination, which are particularly high in perishable goods such as meat, cheese and dairy products in general, fruit, vegetables, etc.

For the same reasons as those described above, the panels 1 can advantageously be used in static storage rooms, operating rooms, dental surgeries and in any place where bacterial contamination constitutes a real risk.

Below, by way of example, are the results of experiments conducted to demonstrate bacterial reduction efficacy over irradiation time, in particular for reducing the charge of the following bacterial strains: Escherichia coli, Bacillus subtilis, Staphylococcus aureus, Pseudomonas aeruginosa.

The antibacterial property was assessed through analysis of the aforesaid bacterial strains according to ISO 27447:2009 (AATCC 100:2004 for the preparation of the inoculants).

The table below shows the antibacterial activity of the panel 1 assessed for each bacterial strain.

### REDUCTION RATE (%) ASSESSED AFTER 3h AND 24h FROM IRRADIATION

| SAMPLE | Escherichia Coli | | Bacillus Subtilis | | Pseudomonas aeruginosa | | Staphylococcu s aureus | |
|---|---|---|---|---|---|---|---|---|
| | 3h | 24h | 3h | 24h | 3h | 24h | 3h | 24h |
| PANEL irradiated (380 nm) for 1h | 95.30 | 0 | 76.50 | - | 92.90 | - | 57.90 | - |

Analysis 24h after inoculation of Escherichia Coli confirmed that titanium dioxide was no longer active after such a long time.

A pool of the four bacterial strains was then analysed by taking samples at 0, 1, 3, 5 and 7 hours.

| **PANEL (first embodiment) irradiated (380 nm) for 1h** | **Incubation time (h) after irradiation** | **Reduction rate (%)** |
|---|---|---|
| | 0 | 0.00 |
| | 1 | 45.00 |
| | 3 | 70.80 |
| | 5 | 0.00 |
| | 7 | 0.00 |
| PANEL (second embodiment) irradiated (380 nm) | Incubation time (h) after irradiation | Reduction rate (%) |
| With chips | 0 | 0.00 |
| Without chips | 0 | 0.00 |
| With chips | 3 | 64.60 |
| Without chips | 3 | 75.00 |

It should be noted that the anti-bacterial panel 1 is effective against the bacterial pool in question for up to 3h after inoculation (reduction rate of 70%) in the case of both the first embodiment (Figure 1) and the second embodiment (Figure 2). Thus, considering that 1 hour passes between the end of irradiation and inoculation of the bacterial pool, it may be said that the panel 1, after irradiation for 60 minutes, presents excellent antibacterial properties for a length of time of 4 hours.

Further developments of the experimental tests conducted demonstrated that the panels 1 were also highly effective for inactivating certain viral strains.

More specifically, by way of example, the efficacy of the panels 1 for inactivating the following viral strains was demonstrated:
Flu virus type A(H1N1) 2009;
Adenovirus type 2;
Herpes simplex virus type 1;
Vaccine poliovirus type 1.

The above viruses were cultured and titrated in TCID50 (doses infecting 50% of the cell cultures).

The results of the tests performed are summarised in Table 1 below.

The TCID₅₀ flu virus recovered at time 0 corresponds to the concentration of the virus eliminated from patients with flu on the first day of infection.

**Table 1 - Persistence of viral infectivity on tiles T (treated = photocatalytic) and NT (untreated = non photocatalytic) at different times after contamination with 2009 pandemic flu virus, vaccine poliovirus ype 1, human Herpes virus type 1 and human Adenovirus type 2 (results expressed in log TCID₅₀±SD)**

| | | **log TCID₅₀±SD after an irradiation time of:** | | | | | | | |
|---|---|---|---|---|---|---|---|---|---|
| *Virus* | | *0 min* | *30 min* | *1 hour* | *2 hours* | *4 hours* | *8 hours* | *16 hours* | *24 hours* |
| H1N1 2009 | T | 5.2±0.2 6 | 2.50±0.2 5 | 1.25±0.2 0 | 0 | | | | |
| | NT | | 4.25±0.3 8 | 4.25±0.3 8 | 2.25±0.2 3 | | | | |
| PV 1 | T | 5.5±0.4 3 | 1.5±0.25 | 1.5±0.25 | 0 | | | | |
| | NT | | 4.5±0.43 | 4.5±0.43 | 3.25±0.2 3 | | | | |
| HSV 1 | T | 5.33±0. 14 | | | 3.5±0.25 | 1.5±0.27 5 | 1.25±0.2 0 | 1.25±0.2 0 | 0 |
| | NT | | | | 5.25±0.2 0 | 4.00±0.2 0 | 3.25±0.2 0 | 3.25±0.2 0 | 1.25±0.2 0 |
| ADV 2 | T | 5.2±0.1 3 | | | - | 2.75±0.3 7 | 1.75±0.3 7 | 1.62±0.2 2 | 0 |
| | NT | | | | - | 4.5±0.25 | 3.25±0.2 5 | 3.25±0.2 0 | 1.75±0.3 7 |

Table 1 shows that on the treated tiles exposed to the light of the lamp, the presence of the flu virus was considerably reduced after just one hour and no longer detectable after 2 hours. On the untreated tiles NT, the infecting flu virus persisted after 8 hours and after 16 hours from contamination. The behaviour of vaccine poliovirus type 1 was not very different. In this case, too, a sharp reduction in the infecting charge was noted after 30 minutes and the virus was no longer recoverable after the contaminated tiles T had been exposed to the light for two hours.

A different behaviour was observed with herpes virus type 1 and with adenovirus type 2 which, after exposure to the light of the lamp for 2, 4, 8 and 16 hours persisted on the tiles T and, obviously, also on the tiles NT. It should, however, be noted that after exposure to the light for just 4 hours, the residual viral charge on the tiles T, especially, but not only, with regard to HSV 1 (almost 4 log), but also with regard to ADV 2 (almost 3 log), was considerably reduced. Complete elimination of the virus from the contaminated surfaces was observed after the tiles T had been exposed to the light of the lamp for 24 hours. Also in the case of herpes virus and adenovirus, the viral charge on all the contaminated tiles was considerably reduced after 24 hours.

Moreover, it should be considered that the panel 1 is activated quickly and automatically by the light source 9.

Thus, with reference, for example, to the ambulance 8, the sanitary compartment 8a need not be manually sanitised by the personnel in charge.

In alternative, simplified embodiments, the light source for catalysing the antibacterial covering panels 1 is not mounted permanently in the compartment but may be installed therein temporarily for the length of time needed for irradiation of the panels 1 themselves.

In the case of goods transport with the van 10, the respective compartment 10a may be sanitised by irradiation before the goods are loaded into it, considering that the goods will remain in it for a length of time which is shorter than the estimated time taken by the panels 1 to lose their effective photocatalytic action.

## Claims

1. A method for making an anti-bacterial covering panel, comprising the steps of:
- preparing a mould (S);
- associating with the mould (S) at least one composite material (3) made by applying at least one layer (4) of particles of photocatalytic material;
- associating with the composite material a supporting layer (2);
**characterized in that** the step of associating at least one composite material (3) comprises the sub-steps of:
- distributing a plurality of superposed layers (4) of particles of photocatalytic material; and
- distributing on each layer (4) of particles of photocatalytic material a respective layer (5) of resin by spraying the resin onto the layer (4) of particles of photocatalytic material.

2. The method according to the preceding claim, **characterised in that** the step of applying a layer (4) of photocatalytic material comprises the sub-step of spraying an alcoholic solution of nanoparticle titanium dioxide on the mould (S).

3. The method according to claim 2, **characterized in that** the step of applying a layer (4) of photocatalytic material is carried out in such a way that the layer (4) of particles of photocatalytic material defines a nanometer thickness.

4. The method according to any of the preceding claims, **characterized in that** it comprises, next to the step of distributing said at least one layer (5) of resin, a step of applying a supporting layer (2) to the last resin layer (5).

5. The method according to claim **4**, **characterized in that** it comprises a step of removing the mould (S) once a cross-linking of the supporting layer (2) has taken place, in order to obtain the panel (1).

6. The method according to any of the preceding claims, **characterized in that** the resin is polyester.

7. The method according to any of claims from 1 to 5, **characterized in that** each resin layer (5) is gelcoat.

8. The method according to claim 7, **characterized in that** said gelcoat contains colour pigments to give the panel (1) a certain aesthetic appearance.

## Patentansprüche

1. Verfahren zur Herstellung einer antibakteriellen Verkleidungsplatte, umfassend die folgenden Schritte:
- Vorbereiten eines Formwerkzeugs (S);
- In-Verbindung-Bringen mindestens eines Verbundmaterials (3) mit dem Formwerkzeug (S), das durch Aufbringen mindestens einer Schicht (4) aus Partikeln photokatalytischen Materials hergestellt wird;
- In-Verbindung-Bringen einer Trägerschicht (2) mit dem Verbundmaterial;
**dadurch gekennzeichnet, dass** der Schritt des In-Verbindung-Bringens des mindestens einen Verbundmaterials (3) die folgenden Unterschritte umfasst:
- Verteilen einer Vielzahl von übereinanderliegenden Schichten (4) aus Partikeln photokatalytischen Materials; und
- Verteilen auf jeder Schicht (4) aus Partikeln photokatalytischen Materials eine jeweilige Schicht (5) aus Harz durch Aufsprühen des Harzes auf die Schicht (4) aus Partikeln photokatalytischen Materials.

2. Verfahren nach dem vorangehenden Anspruch, **dadurch gekennzeichnet, dass** der Schritt des Aufbringens einer Schicht (4) aus photokatalytischem Material den Unterschritt des Aufsprühens einer alkoholischen Lösung aus Nanopartikeln Titandioxid auf das Formwerkzeug (S) umfasst.

3. Verfahren nach Anspruch 2, **dadurch gekennzeichnet, dass** der Schritt des Aufbringens einer Schicht (4) aus photokatalytischem Material so durchgeführt wird, dass die Schicht (4) aus Partikeln photokatalytischen Materials eine Nanometerdicke definiert.

4. Verfahren nach einem der vorangehenden Ansprüche, **dadurch gekennzeichnet, dass** es neben dem Schritt des Verteilens der mindestens einen Schicht (5) aus Harz einen Schritt des Aufbringens einer Trägerschicht (2) auf die letzte Harzschicht (5) umfasst.

5. Verfahren nach Anspruch 4, **dadurch gekennzeichnet, dass** es einen Schritt des Entfernens des Formwerkzeugs (S) umfasst, sobald die Vernetzung der Trägerschicht (2) stattgefunden hat, um die Platte (1) zu erhalten.

6. Verfahren nach einem der vorangehenden Ansprüche, **dadurch gekennzeichnet, dass** das Harz Polyester ist.

7. Verfahren nach einem der Ansprüche 1 bis 5, **dadurch gekennzeichnet, dass** jede Harzschicht (5) Gelcoat ist.

8. Verfahren nach Anspruch 7, **dadurch gekennzeichnet, dass** das Gelcoat Farbpigmente enthält, um der Platte (1) ein bestimmtes ästhetisches Aussehen zu verleihen.

## Revendications

1. Procédé de fabrication d'un panneau de revêtement antibactérien, comprenant les étapes de :
- préparer un moule (S) ; ;
- associer au moule (S) au moins un matériau composite (3) réalisé en appliquant au moins une couche (4) de particules de matériau photocatalytique ;
- associer au matériau composite une couche de support (2) ;
**caractérisé en ce que** l'étape consistant à associer au moins un matériau composite (3) comprend les sous étapes de :
- répartir une pluralité de couches superposées (4) de particules de matériau photocatalytique ; et
- répartir sur chaque couche (4) de particules de matériau photocatalytique une couche respective (5) de résine en pulvérisant la résine sur la couche (4) de particules de matériau photocatalytique.

2. Procédé selon la revendication précédente, **caractérisé en ce que** l'étape consistant à appliquer une couche (4) de matériau photocatalytique comprend la sous étape consistant à pulvériser une solution alcoolique de nanoparticules de dioxyde de titane sur le moule (S).

3. Procédé selon la revendication 2, **caractérisé en ce que** l'étape consistant à appliquer une couche (4) de matériau photocatalytique est effectuée de manière à ce que la couche (4) de particules de matériau photocatalytique définisse une épaisseur de l'ordre du nanomètre.

4. Procédé selon l'une quelconque des revendications précédentes, **caractérisé en ce qu'**il comprend, après l'étape consistant à répartir ladite au moins une couche (5) de résine, une étape consistant à appliquer une couche de support (2) à la dernière couche de résine (5).

5. Procédé selon la revendication 4, **caractérisé en ce qu'**il comprend une étape consistant à retirer le moule (S) dès qu'une réticulation de la couche de support (2) a eu lieu, afin d'obtenir le panneau (1).

6. Procédé selon l'une quelconque des revendications précédentes, **caractérisé en ce que** la résine est du polyester.

7. Procédé selon l'une quelconque des revendications de 1 à 5, **caractérisé en ce que** chaque couche de résine (5) est de l'enduit gélifié.

8. Procédé selon la revendication 7, **caractérisé en ce que** ledit enduit gélifié contient des pigments colorés pour donner un certain aspect esthétique au panneau (1).
